(19) Europäisches Patentamt
European Patent Office
Office européen des brevets

(11) Numéro de publication: **0 211 774**
**A2**

# (12) DEMANDE DE BREVET EUROPEEN

(21) Numéro de dépôt: **86401854.4**

(22) Date de dépôt: **21.08.86**

(51) Int. Cl.[4]: **A61K 31/40** , A61K 31/535 , A61K 31/495

(30) Priorité: **22.08.85 FR 8512619**

(43) Date de publication de la demande: **25.02.87 Bulletin 87/09**

(84) Etats contractants désignés: **AT BE CH DE FR GB IT LI LU NL SE**

(71) Demandeur: **ROUSSEL-UCLAF**
**35, boulevard des Invalides**
**F-75007 Paris(FR)**

(72) Inventeur: **Delevallee, Françoise**
**48-50 Avenue de la Dame Blanche**
**F-94120 Fontenay Sous Bois(FR)**
Inventeur: **Deraedt, Roger**
**23, Allée Jean-Baptiste Clément**
**F-93320 Les Pavillons Sous Bois(FR)**
Inventeur: **Benzoni, Josette**
**11, Boulevard Max Dormoy**
**F-93190 Livry-Gargan(FR)**

(74) Mandataire: **Fritel, Hubert et al**
**Département des Brevets ROUSSEL UCLAF**
**B.P. no 9**
**F-93230 Romainville(FR)**

## (54) Utilisation de dérivés de l'acide maléopimarique pour l'obtention d'un medicament immunomodulateur.

(57) L'invention concerne l'utilisation de dérivés de l'acide maléopimarique de formule :

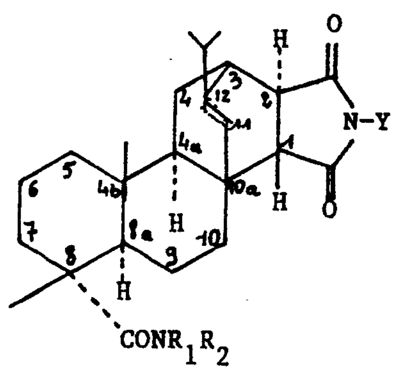

($R_1R_2N$ = hétérocycle saturé comprenant éventuellement d'autres hétéroatomes et éventuellement substitué ;

Y = H ; $NH_2$ ; alkyle $C_1C_5$ éventuellement substitué par OH, dialkylamino ou alkloxycarbonyle ; acyle ; -XO-CO-CO-$NR_1R_2$ où $R_1R_2N$ a la signification

EP 0 211 774 A2

précitée et X = alcoylène), pour l'obtention d'un médicament immunomodulateur.

**Utilisation de dérivés de l'acide maléopimarique pour l'obtention d'un médicament immunomodulateur.**

La présente invention a pour objet l'utilisation de dérivés de l'acide de maléopimarique pour l'obtention d'un médicament immunomodulateur.

Le brevet français n¤ 2.202.691 a décrit de nouveaux dérivés de l'acide maléopimarique, leur procédé de préparation et leur application à titre de médicaments. Ces composés ont été présentés comme hépatoprotecteurs.

La demanderesse, en poursuivant les recherches sur cette série, a découvert de manière tout à fait inattendue, que ces dérivés étaient de plus doués de remarquables propriétés immunomodulatrices ; propriétés qui, à la connaissance de la demanderesse, n'ont aucun rapport ou lien de parenté avec les propriétés hépatoprotectrices.

Par immunomodulateur, on entend "qui règle les réactions immunitaires en les inhibitant ou en les stimulant" (MM. GARNIER -DELAMARE -Dictionnaire des termes techniques de Médecine p.421 -21ème édition 1985).

C'est pourquoi la présente demande a pour objet l'utilisation des dérivés de l'acide maléopimarique de formule (I)

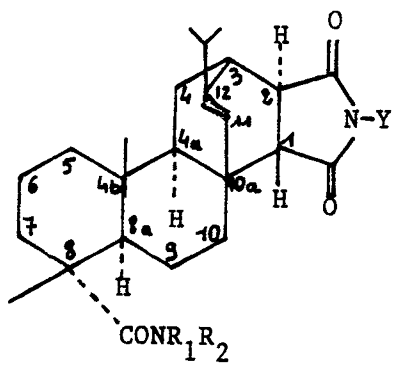

(I)

dans laquelle $R_1$ et $R_2$ forment ensemble avec l'atome d'azote un hétérocycle saturé pouvant comporter un ou plusieurs autres hétéroatomes, et qui pouvant substitué ou non substitué, et Y représente un atome d'hydrogène, un groupe amino, un groupe alcoyle pouvant être substitué par un ou plusieurs groupes hydroxy, dialcoylamino ou alcoyloxycarbonyle, ou Y représente un groupe acyle ou un groupe de formule :

$-XO-CO-CO-NR_1R_2$

dans laquelle $R_1$ et $R_2$ ont la signification précitée, et X représente un groupe alcoylène, tous les groupes alcoyle, alcoylène et acyle comportant au plus 5 atomes de carbone, pour l'obtention d'un médicament immunomodulateur.

$R_1$ et $R_2$, ensemble avec l'atome d'azote, représentent, par exemple, un groupe hétérocyclique saturé comportant de 4 à 6 atomes de carbone, tel que, par exemple, un groupe morpholino, pipéridino, pipérazin-1-yl, pyrrolidino ou hexaméthylène imino, ce groupe pouvant porter un substituant soit sur un atome de carbone, le substituant étant alors un alcoyle, un hydroxyalcoyle ou un phényle, soit sur un atome d'azote, le substituant étant alors un phényle, un alcoyloxycarbonyle, un sulfonyle ou un acyle. De tels groupes alcoyle, alcoyloxy ou acyle comportent au plus 5 atomes de carbone.

Y représente, par exemple, un groupe hydroxyalcoyle, un groupe dialcoylaminoalcoyle ou un groupe alcoyloxycarbonyle, les groupes alcoyle comportant de 1 à 5 atomes de carbone.

Les composés préférés, selon l'invention, sont ceux de formule I dans laquelle $R_1$ et $R_2$, ensemble avec l'atome d'azote, représentent un groupe pyrrolidino, morpholino, pipérazin-1-yl, 4-alcoyl pipérazin-1-yl ou 4-hydroxyalcoyl-pipérazin-1-yl et ceux pour lesquels Y représente un atome d'hydrogène, un groupe amino ou un groupe $\beta$-hydroxyéthyle et tout particulièrement ceux de formule -(I) dans laquelle $R_1$ et $R_2$, ensemble avec l'atome d'azote, représentent un groupe morpholino et ceux pour lesquels Y représente un atome d'hydrogène ou un groupement $\beta$-hydroxy éthyle.

Les composés de formule I particulièrement préférés pour leur utilisation pour l'obtention de médicaments immunomodulateurs sont la 8 - morpholinocarbonyl-4b α,8α-diméthyl-12-isopropyl-1β,2β,3β,4,4aβ-4b α,5,6,7,8,8aβ,9,10,10a-tétradécahydro-3,10a-éthénophénantro-/1,2-c/1'-(2-hydroxy éthyl)-2',5'-pyrrolidinedione et la 8β-(morpholino carbonyl)-4bα,8α-diméthyl-12-isopropyl-1β,2β,3β,4,4aβ,4bα,5,6,7,8, 8aβ,9,10,10a-tétradécahydro-3,10a-éthénophénanthro-/1,2 c/2',5'-pyrrolidinedione.

Les dérivés de formule I possèdent de remarquables propriétés immunomodulatrices illustrées plus loin dans la partie expérimentale. Ils se distinguent de plus par une très faible toxicité et une remarquable tolérance.

En raison de ces propriétés, les médicaments immunomodulateurs renfermant ces dérivés peuvent être administrés à titre préventif ou curatif. Ils trouvent leur emploi dans le traitement des maladies auto-immunes, qu'il s'agisse d'atteintes non spécifiques d'organes (polyarthrite rhumatoïde, lupus érythémateux, anémie hémolytique, leucopénie auto-immune etc ...) ou qu'il s'agisse de maladies spécifiques d'organes (thyroïdite, maladie de Basedow, d'Addison, sclérose en plaques, pemphigus, rectocolite hémorragique, certaines néphropathies etc ...). Ces médicaments peuvent également être utilisés dans le traitement des hémopathies, du cancer, du sida, des affections virales et microbiennes, surtout chroniques et recurrentes (bronchite, grippe etc ...) des maladies des cavités buccales, etc ... Ils peuvent constituer des adjuvants de la thérapie virale, de l'antibiothérapie ou de la chimiothérapie anticancéreuse.

Ils trouvent également leur application dans le traitement de nombreux déficits immunitaires secondaires ou acquis observés au cours d'affections très diverses : déficits associés aux troubles métaboliques, déficits d'origine iatrogène (corticoïdes, radiations ionisantes ...) déficits observés chez les grands brûlés, etc ...

Les dérivés de formule (I) peuvent être utilisés pour préparer des compositions pharmaceutiques et celles-ci peuvent se présenter avantageusement en doses unitaires contenant par unité 25 à 500 mg de composé de formule (I).

La dose journalière varie entre 25 mg et 3 g selon la nature de la maladie, le malade traité et la voie utilisée.

Dans le cas d'utilisation des médicaments renfermant ces dérivés comme traitement adjuvant de l'antibiothérapie ou de la thérapie antivirale, la durée de traitement sera par exemple égale ou supérieure à celle de l'antibiothérapie ou de la thérapie antivirale.

Dans les autres cas, l'administration sera prolongée sur une longue période (par exemple 3 mois à 2 ans ou plus) et pourra se faire de manière discontinue.

Ces médicaments immunomodulateurs peuvent être destinés à la voie digestive, parentérale, ou à une application locale.

Ces médicaments peuvent être par exemple, solides ou liquides et se présenter sous les formes pharmaceutiques couramment utilisées en médecine humaine, comme par exemple les comprimés simples ou dragéifiés, les gélules, les capsules, les granulés, les préparations injectables, les gels, les pommades et les crèmes.

Les dérivés de formule (I) peuvent être incorporés à des excipients habituellement employés dans ces compositions pharmaceutiques tels que le talc, lagomme arabique, le lactose, l'amidon, le stéarate de magnésium, le beurre de cacao, les véhicules aqueux ou non, les corps gras d'origine animale ou végétal, les dérivés paraffiniques, les glycols, les divers agents mouillants, dispersants ou émulsifiants, les conservateurs.

Les dérivés de l'acide maléopimarique répondant à la formule générale I peuvent être préparés comme indiqué, par exemple dans le brevet français N¤ 2.202.691.

Les exemples suivants illustrent l'invention sans toutefois la limiter.

EXEMPLE 1 :

On a préparé des comprimés répondant à la formule :

-8β-morpholino-carbonyl-4bα, 8α-diméthyl-12-isopropyl-(1β,2β,3β, 4, 4aβ,4bα, 5,6,7,8,8aβ,9,10,10a-tétradécahydro-3,10a-éthénophénanthro /1,2-c/-1'-(2-hydroxy-éthyl)-2',5'-pyrrolidinedione ...................... 200 mg

Excipient : quantité suffisante pour 1 comprimé terminé à ..... 300 mg.

(Détail de l'excipient : lactose, amidon, talc, stéarate de Mg).

EXEMPLE 2 :

On a préparé des comprimés répondant à la formule :

-8β-morpholinocarbonyl-4bα,8α-diméthyl-12-isopropyl-1β,2β,3β,4,4aβ,4bα, 5,6,7,8,8aβ,9,10,10a-tétradécahydro-3,10a-éthénophénanthro-/1,2-c/-2',5'-pyrrolidinedione (maléopimarimidyl)

.............................. 200 mg.

Excipient : quantité suffisante pour 1 comprimé terminé à ....... 300 mg.

(Détail de l'excipient : lactose, amidon, talc, stéarate de Mg).

EXEMPLE 3 :

On a préparé un soluté injectable répondant à la formule :

-8β-morpholino-carbonyl-4bα,8α-diméthyl-12-isopropyl (1β,2β,3β,4,4aβ,4bα,5,6,7,8,8aβ,9,10,10a-tétradécahydro-3,10a-éthenénophénanthro /1,2-c/-1'-(2-hydroxy-éthyl)-2',5'-pyrrolidinedione ...................... 150 mg

Solvant aqueux : quantité suffisante pour 2 cm3.

EXEMPLE 4 :

On a préparé un soluté injectable répondant à la formule :

-8β-morpholinocarbonyl-4bα,8α-diméthyl-12-isopropyl-1β,2β,3β,4,4aβ,4bα,5,6,7,8,8aβ,9,10,10a-tétradécahydro-3,10a-éthénophénanthro-/1,2-c/-2',5'-pyrrolidinedione ............................................ 150 mg

Solvant aqueux : quantité suffisante pour 2 cm3

EXEMPLE 5 :

On a préparé des gélules répondant à la formule suivante :

-8β-morpholino-carbonyl-4bα,8α-diméthyl-12-isopropyl (1β,2β,3β,4,4aβ,4bα,5,6,7,8,8aβ,9,10,10a-tétradécahydro-3,10a-éthénophénanthro /1,2-c/-1'-(2-hydroxy-éthyl)-2',5'-pyrrolidinedione .................... 200 mg.

Excipients : (mannite, acide citrique, chlorure de sodium, thiourée, éthylène diaminetétracétate de sodium, lactose, méthylcellulose, stéarate de magnésium) quantité suffisante pour une gélule.

EXEMPLE 6 :

On a préparé une pommade répondant à la formule suivante :

- 8β-morpholino-carbonyl-4bα,8α-diméthyl-12-isopropyl (1β,2β,3β,4,4aβ,4bα,5,6,7,8,8aβ,9,10,10a-tétradécahydro-3,10a-éthénophénanthro /1,2-c/-1'-(2-hydroxyéthyl)-2',5'pyrrolidinedione .................... 1,5 g.

Excipients : (alcool céthylique, triglycérides végétaux saturés, esters de polyéthylène glycol 2000 avec des acides gras en $C_{12}$à $C_{14}$, Tween 80, para-oxy-benzoates, sorbitol, polymère carboxyvinylique, sulfite de sodium, triéthanolamine, lécithine, eau purifiée, acide lactique) quantité suffisante pour ......... 100 g.

EXEMPLE 7 :

On a préparé une crème répondant à la formule suivante :

-8β-morpholino-carbonyl-4bα,8α-diméthyl-12-isopropyl (1β,2β,3β,4,4aβ,4bα,5,6,7,8,8aβ,9,10,10a-tétradécahydro-3,10a-éthénophénanthro /1,2-c/- 1'-(2-hydroxy-éthyl)-2',5'-pyrrolidinedione ......................... 1,5 g

Excipients : (alcool 2 octyl 2-dodécanol, alcool cétostéarylique, cétostéaryl sulfate de sodium, parahydroxybenzoate de méthyle et de propyle, eau purifiée), quantité suffisante pour ............ 100 g.

ETUDE PHARMACOLOGIQUE

Cette étude a été faite sur les produits suivants :

PRODUIT A

8β-morpholino-carbonyl-4bα,8α-diméthyl-12-isopropyl(1β,2β,3β,4,4aβ,4bα,5,6,7,8,8aβ,9,10,10a-tétradécahydro-3,10a-éthénophénanthro/1,2-c/-1'-(2-hydroxy-éthyl)-2',5'-pyrrolidinedione.

PRODUIT B

8β-morpholinocarbonyl-4bα,8α-diméthyl-12-isopropyl-1β,2β,3β,4 ,4aβ,4bα,5,6,7,8,8aβ,9,10,10a-tétradécahydro-3,10a-éthénophénanthro/1,2-c/-2',5'-pyrrolidinedione.

1 -Test des rosettes aux globules rouges de moutons

L'administration a des animaux d'un produit capable de stimuler l'activité des systèmes immunitaires se traduit par une augmentation de leur capacité de réaction à l'injection d'un produit immunogène.

Des rats mâles âgés de 3 mois sont sensibilisés par voie intrapéritonéale avec des érythrocytes de mouton (jour 0). 7 jours plus tard (Jour 7), leur rate est prélevée et les splénocytes sont mis en contact avec des érythrocytes de mouton. On compte ensuite le pourcentage de leucocytes autour desquels les érythrocytes ont formé des rosettes.

Le produit à étudier est administré per os quotidiennement du jour -1 au jour 1.

On considère comme dose immunostimulante la dose de produit qui multiplie environ par 2 le pourcentage de rosettes observé chez les animaux témoins.

Les doses actives pour les produits A et B sont respectivement de 1 et 2 mg/kg par voie buccale.

2 -Test de formation d'anticorps : détermination du taux d'hémagglutinine chez la souris.

Des souris reçoivent une administration intraveineuse de globules rouges de moutons le premier jour. Le produit est administré à la dose de 100 mg/kg per os du 1er au 3ème jour. Le quatrième jour, les animaux sont sacrifiés et les titres en anticorps sériques sont déterminés par hémagglutination.

On observe avec le produit A une augmentation de 23% du titre par rapport à celui des animaux témoins.

3 -Toxicité aiguë

Les produits testés sont très bien tolérés chez les rongeurs après administration orale aiguë. En effet, pour le produit A, les DL 50 sont respectivement de 10 g/kg et 6,3 g/kg chez la souris et le rat.

## Revendications

1/. Utilisation des dérivés de l'acide maléopimarique de formule I:

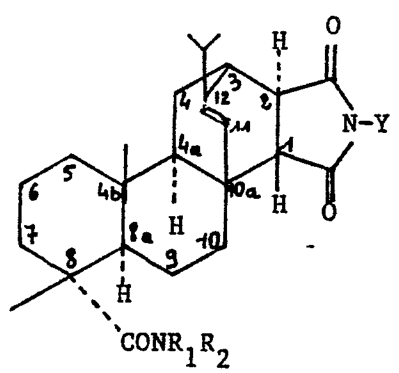

(I)

dans laquelle $R_1$ et $R_2$ forment ensemble avec l'atome d'azote un hétérocycle saturé pouvant comporter un ou plusieurs autres hétéroatomes, et pouvant être substitué ou non substitué, et Y représente un atome d'hydrogène, un groupe amino, un groupe alcoyle pouvant être substitué par un ou plusieurs groupes hydroxy, dialcoylamino ou alcoyloxycarbonyle, ou Y représente un grouple acyle ou un groupe de formule :

$-XO-CO-CO-NR_1R_2$

dans laquelle $R_1$ et $R_2$ ont la signification précitée, et X représente un groupe alcoylène, tous les groupes alcoyle, alcoylène et acyle comportant au plus 5 atomes de carbone, pour l'obtention d'un médicament immunomodulateur.

2/. Utilisation des dérivés selon la revendication 1 dans lesquels $R_1$ et $R_2$, ensemble avec l'atome d'azote, représentent un hétérocycle saturé comportant de 4 à 6 atomes de carbone, pour l'obtention d'un médicament immunomodulateur.

3/. Utilisation des dérivés selon la revendication 2, dans lesquels $R_1$ et $R_2$, ensemble avec l'atome d'azote, représentent un groupe morpholino, pipéazin-1-yle, pyrrolidino, 4-alcoylpipérazin-1-yl ou 4-hydroxyalcoyl pipérazin-1-yl et Y représente un atome d'hydrogène, un groupe amino ou un groupe$\beta$ -hydroxyéthyle, pour l'obtention d'un médicament immunomodulateur.

4/. Utilisation des dérivés selon la revendication 3, dans lesquels $R_1$ et $R_2$, ensemble avec l'atome d'azote représentent un groupement morpholino, pour l'obtention d'un médicament immunomodulateur.

5/. Utilisation des dérivés selon l'une des revendications 1 à 4, dans lesquels Y représente un atome d'hydrogène ou un groupement$\beta$-hydroxy éthyle, pour l'obtention d'un médicament immunomodulateur.

6/. Utilisation de la 8$\beta$-morpholino-carbonyl-4b$\alpha$, 8$\alpha$-diméthyl-12-isopropyl 1$\beta$,2$\beta$,3$\beta$,4,4a$\beta$,4b$\alpha$,5,6,7,8,8a$\beta$,9,10,10a-tétradécahydro-3,10a-éthénophénanthro /1,2-c/-1'-(2-hydroxy-éthyl)-2'5'-pyrrolidinedione, pour l'obtention d'un médicament immunomodulateur.

7/. Utilisation de 8$\beta$-morpholinocarbonyl-4 b$\alpha$,8$\alpha$-diméthyl-12-isopropyl-1$\beta$,2$\beta$,3$\beta$,4,4a$\beta$,4b$\alpha$,5,6,7,8,8a$\beta$,9,10,10a-tétradécahydro-3,10a-éthénophénanthro-/1,2-c/-2',5'-pyrrolidinedione, pour l'obtention d'un médicament immunomulateur.